(19) Europäisches Patentamt
European Patent Office
Office européen des brevets

(11) EP 2 418 276 B1

(12) EUROPÄISCHE PATENTSCHRIFT

(45) Veröffentlichungstag und Bekanntmachung des Hinweises auf die Patenterteilung:
**20.11.2013 Patentblatt 2013/47**

(51) Int Cl.:
***C12N 15/00*** *(2006.01)* ***C12N 15/67*** *(2006.01)*
***C12N 1/00*** *(2006.01)* ***C12N 1/20*** *(2006.01)*

(21) Anmeldenummer: **10175512.2**

(22) Anmeldetag: **29.01.2007**

(54) **Mikroorganismenstamm zur Produktion von rekombinanten Proteinen**

Microorganism strain for producing recombinant proteins

Procédé destiné à la fabrication d’une broche

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **02.02.2006 DE 102006004871**

(43) Veröffentlichungstag der Anmeldung:
**15.02.2012 Patentblatt 2012/07**

(62) Dokumentnummer(n) der früheren Anmeldung(en) nach Art. 76 EPÜ:
**07101292.6 / 1 897 939**

(73) Patentinhaber: **Wacker Chemie AG**
**81737 München (DE)**

(72) Erfinder:
- **Leonhartsberger, Susanne, Dr.**
  **80639 München (DE)**
- **Wich, Günter, Dr.**
  **81377 München (DE)**

(74) Vertreter: **Potten, Holger et al**
**Wacker Chemie AG**
**Zentralbereich Patente, Marken und Lizenzen**
**Hanns-Seidel-Platz 4**
**81737 München (DE)**

(56) Entgegenhaltungen:

- **OLSON E R ET AL: "Identification and characterization of dppA, an Escherichia coli gene encoding a periplasmic dipeptide transport protein", JOURNAL OF BACTERIOLOGY, AMERICAN SOCIETY FOR MICROBIOLOGY, WASHINGTON, DC; US, Bd. 173, Nr. 1, 1. Januar 1991 (1991-01-01), Seiten 234-244, XP002991770, ISSN: 0021-9193**
- **LESSARD IAD ET AL: "VanX, a bacterial D-alanyl-D-alanine dipeptidase: Resistance, immunity, or survival function?", PROCEEDINGS OF THE NATIONAL ACADEMY OF SCIENCES USA, vol. 96, 1 September 1999 (1999-09-01), pages 11028-11032,**

Bemerkungen:

Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 418 276 B1

## Beschreibung

Die Erfindung betrifft Mikroorganismenstämme, die eine verbesserte Herstellung von rekombinanten Proteinen ermöglichen, Verfahren zur Herstellung derartiger Stämme und Verfahren zur Herstellung von rekombinanten Proteinen unter Einsatz dieser Stämme.

Die großvolumige wirtschaftliche Herstellung von rekombinanten Proteinen ist für die biotechnologische und pharmazeutische Industrie von wachsender Bedeutung. Generell werden rekombinante Proteine entweder in Säugerzellkulturen oder in mikrobiellen Systemen hergestellt. Mikrobielle Systeme weisen gegenüber Säugerzellkulturen den Vorteil auf, dass auf diese Weise rekombinante Proteine in kürzerer Zeit und zu geringeren Kosten hergestellt werden können. Das Bakterium E. coli ist der für die Produktion von rekombinanten Proteinen am weitesten verbreitete mikrobielle Organismus. In E. coli können Proteine prinzipiell auf verschiedene Arten hergestellt werden:

1. intrazelluläre Produktion als lösliches Protein;
2. intrazelluläre Produktion als Einschlusskörperchen ("inclusion bodies");
3. Sekretion in das Periplasma oder in das umgebende Nährmedium.

Aufwand und Kosten der Herstellung des erwünschten Proteins werden neben den Produktionskosten des Rohproduktes, welches direkt nach der Fermentation als Gemisch umfassend das rekombinante Protein sowie Wirtsproteine, die von der Zelle natürlicherweise sekretiert werden, vorliegt, auch wesentlich bestimmt durch die Aufreinigungskosten des Rohproduktes zum erwünschten Protein. Die Aufreinigung erfolgt in den meisten Fällen über mehrere Stufen mittels chromatographischer Verfahren. Dabei spielt die Abreinigung von verunreinigenden Wirtsproteinen, die z. T. immunogen oder toxisch sind, eine bedeutende Rolle.

Die Sekretion von Proteinen in E. coli erfolgt in den meisten Fällen über den sogenannten sec-pathway. Dafür wird das Gen für das zu produzierende Protein mit einer Signalsequenz verknüpft, was zu einer Produktion einer Signalpeptid-Protein-Fusion führt. Das Signalpeptid vermittelt die Sekretion des Proteins über die Cytoplasma-Membran in das Periplasma durch das bakterieneigene sec-System. Dabei wird die Signalsequenz abgespalten und das gewünschte Protein im Periplasma erhalten. Das Protein kann danach aus dem Periplasma gereinigt werden. Unter bestimmten Bedingungen oder in bestimmten BakterienStämmen wird das Protein aus dem Periplasma in das umgebende Nährmedium freigesetzt (z. B. Ray et al. 2002; EP0338410B1; Nagahari et al. 1985; Yang et al., 1998) und kann aus diesem aufgereinigt werden.

Die Sekretion bietet im Vergleich zu den anderen Herstellungsverfahren den Vorteil, dass natives, lösliches, korrekt gefaltetes Protein erhalten wird, welches im Vergleich zum "inclusion body"-Verfahren nicht denaturiert und wieder renaturiert werden muss; ein Schritt, der mit hohen Verlusten an Ausbeute einhergeht. Außerdem ist das erhaltene Produkt im Vergleich zur intrazellulären, löslichen Produktion mit weniger Wirtsproteinen verunreinigt, da das Periplasma von Bakterien weitaus weniger an Wirtsproteinen enthält, als das Cytoplasma.

Die Sekretion der Proteine in das umgebende Nährmedium bietet, verglichen mit Sekretion ins Periplasma, den weiteren Vorteil, dass das Protein dann in noch reinerer Form vorliegt. Außerdem ist als erster Aufreinigungsschritt keine aufwändige Präparation des Periplasmas nötig, sondern eine viel einfachere und reproduzierbar verlaufende Abtrennung der ganzen Zellen.

Bei der Herstellung von Proteinen durch Sekretion ist das Rohprodukt insgesamt mit weniger Wirtsproteinen verunreinigt als bei der intrazellulären Produktion. Trotzdem spielen auch hier verunreinigende Wirtsproteine eine Rolle, vor allem Wirtsproteine, die auch natürlicherweise vom Bakterium sekretiert werden und dann im Periplasma oder in der äußeren Membran lokalisiert sind. Diese Proteine zeichnen sich dadurch aus, dass ihre Gene natürlicherweise eine Signalsequenz enthalten, welche diese Sekretion vermittelt. Außer der Tatsache, dass diese Wirtsproteine das Rohprodukt verunreinigen, konkurrieren sie mit dem zu produzierenden Protein um die Komponenten des Sekretionsapparates, was zu einer verringerten Sekretion des zu produzierenden Proteins führen kann. Diese Wirtsproteine, die zu einer Verunreinigung des Rohproduktes führen, erfüllen aber natürlich in der Wirtszelle eine physiologische Rolle. Sie können zum Beispiel an Chemotaxis und speziellen Transportvorgängen beteiligt sein.

In der Literatur ist beschrieben, dass durch die Deletion von Genen, welche für periplasmatische Proteasen codieren, z. B. degP, ompT, ptr, die Produktion proteolyseempfindlicher, sekretierter Proteine verbessert werden kann (US5264365; Baneyx & Georgiou, 1991; Wadensten et al., 1991). Dieser Effekt ist auf das Ausschalten der Aktivität der Proteasen zurückzuführen, welche in der Ausgangszelle das produzierte Protein abbauen. Vor allem heterolog produzierte Proteine in Zellen werden oft durch wirtseigene Proteasen abgebaut. Mengenmäßig spielen die Proteasen als Verunreinigungen jedoch keine Rolle, da sie aufgrund ihrer hohen Aktivität und enzymatischen Funktion in der Wirtszelle nur in sehr geringen Mengen produziert werden. Die Deletion dieser Gene hat also keine Auswirkung auf den Reinheitgrad der produzierten Proteine.

WO2004/035792A1 beschreibt die Veränderung von bestimmten Wirtsproteinen (z. B. PhoS/PstS, DppA, MBP, Trx) durch Mutationen in einzelnen Aminosäuren, welche zu einer Veränderung der physikalischen bzw. biochemischen Eigenschaften führen (Isoelektrischer Punkt, Hydrophobizität, Größe). Diese Veränderung der physikalischen bzw. biochemischen Eigenschaften hat zur Folge, dass die resultierenden veränderten verunreinigenden Wirtsproteine nicht mehr mit dem jeweils erwünschten produzierten Protein co-gereinigt werden, da sie sich beispielweise auf einer Chromatographie-Säule unterschiedlich verhalten. Das Verfahren ist nicht für die Produktion von beliebigen Proteinen nutzbar, da die verunreinigenden Wirtsproteine speziell für jedes zu produzierende Protein verändert werden müssen, weil jedes Protein unterschiedliche biochemische Eigenschaften hat. Im Verfahren gemäß WO2004/035792A1 wird trotz Veränderung der verunreinigenden Wirtsproteine ihre Produktion und Funktionalität beibehalten. Der Reinheitsgrad des Rohproduktes des produzierten Proteines ändert sich also nicht, es erleichtert aber jeweils die Abtrennung der verunreinigenden Wirtsproteine vom Protein.

WO98/18946 beschreibt Zellen, die zusätzlich zum zu produzierenden Protein Dsb-Proteine co-exprimieren und eine Deletion im Wildtyp pstS-Gen haben, die aber gleichzeitig eine pstS-Variante exprimieren. Auch hier ist die Menge an verunreinigendem Wirtsprotein also unverändert.

Eine Aufgabe der vorliegenden Erfindung ist die Bereitstellung von Mikroorganismenstämmen, welche die Produktion von rekombinanten Proteinen erleichtern.

Die Aufgabe wird gelöst durch einen Mikroorganismenstamm nach einem der Ansprüche, der ein Gen kodierend ein rekombinantes Protein sowie ein mutiertes Gen kodierend das Wirtsprotein YddS umfasst, dadurch gekennzeichnet, dass das rekombinante Protein in einer Fermentation sekretiert wird und das mutierte Gen kodierend das Wirtsprotein derart mutiert ist, dass es eine im Vergleich zu dem dem mutierten Gen zugrunde liegenden Wildtyp Gen derart verminderte Expression des Wirtsproteins bewirkt, dass das durch das mutierte Gen Kodierte Wirtsprotein YddS in einer Menge produziert und sekretiert wird, die im Vergleich zu der Produktion und Sekretion in einer Wildtypzelle um 100% verringet ist.

Für eine wirtschaftliche Produktion eines Proteins ist es von Vorteil, wenn das produzierte rekombinante Protein schon im Rohprodukt, also direkt nach der Fermentation, wenn es als Gemisch umfassend das rekombinante Protein sowie verunreinigende Wirtsproteine vorliegt, mit möglichst wenigen Wirtsproteinen in möglichst geringer Menge verunreinigt ist. Damit kommt es nämlich zu einer Erhöhung der spezifischen Ausbeute an rekombinantem Protein. Die nachfolgende Aufreinigung des rekombinanten Proteins wird dadurch vereinfacht. Diesen Vorteil bieten die erfindungsgemäßen Bakterienstämme.

Das rekombinante Protein wird in einer Fermentation vorzugsweise in das Periplasma oder in das Fermentationsmedium sekretiert. Besonders bevorzugt wird das rekombinante Protein in das Fermentationsmedium sekretiert.

Bei dem rekombinanten Protein handelt es sich vorzugsweise um ein heterologes Protein.

Bei dem Mikroorganismenstamm handelt es sich um einen Stamm der Art Escherichia coli. Besonders bevorzugt ist ein Escherichia (E.) coli-Stamm, der sich dadurch auszeichnet, dass er nach Fermentation eine höhere Konzentration des rekombinanten Proteins im Periplasma oder ganz besonders bevorzugt im umgebenden Nährmedium aufweist als der Stamm E. coli W3110 (ATCC 27325). Derartige Stämme werden im Folgenden als Sekretionsstämme bezeichnet.

Besonders bevorzugt sind Sekretionsstämme, die nach Fermentation mehr als 50 mg/l, besonders bevorzugt mehr als 100 mg/l, ganz besonders bevorzugt mehr als 200 mg/l des rekombinanten Proteins im umgebenden Nährmedium aufweisen.

Weitere bevorzugte Merkmale eines erfindungsgemäßen Stammes sind im Rahmen seiner Herstellung genannt. Die im Folgenden genannten Merkmale der Ausgangsstämme gelten damit analog auch für einen erfindungsgemäßen Stamm.

Ein erfindungsgemäßer Mikroorganismenstamm wird erhalten durch ein Verfahren nach einem der Ansprüche, bei dem eine Wirtszelle, die mit einem Gen codierend ein rekombinantes Protein transformiert ist, in einer Fermentation zur Herstellung des rekombinanten Proteins eingesetzt wird, wobei die Wirtszelle das rekombinante Protein sowie weitere Wirtsproteine in das Fermentationsmedium sekretiert und so ein Rohprodukt enthaltend das rekombinante Protein sowie weitere Wirtsproteine erhalten wird, die weiteren Wirtsproteine charakterisiert werden und eine Expression eines Gens kodierend eines dieser Wirtsproteine verringert oder verhindert wird.

Vorzugsweise wird das Gen kodierend ein rekombinantes Protein während des Verfahrens zur Verringerung oder Verhinderung der Expression der Wirtsproteine aus dem Mikroorganismenstamm entfernt.

Bei den Wirtszellen, handelt es sich um Zellen des Stammes der Art Escherichia coli. Besonders bevorzugt ist ein Escherichia (E.) coli-Stamm, der sich dadurch auszeichnet, dass er nach Fermentation eine höhere Konzentration des rekombinanten Proteins im Periplasma oder ganz besonders bevorzugt im umgebenden Nährmedium aufweist als der Stamm E. coli W3110 (ATCC 27325).

Besonders bevorzugt sind solche Sekretionsstämme, die nach Fermentation mehr als 10 mg/l, besonders bevorzugt mehr als 50 mg/l, ganz besonders bevorzugt mehr als 100 mg/l des rekombinanten Proteins im umgebenden Nährmedium aufweisen.

Ganz besonders bevorzugt handelt es sich um einen der folgenden E. coli-Stämme:

- BLR: Ray et al. 2002, käuflich erhältlich bei Novagen

- K802 = CGSC* 5610: Yang et al., 1998
- WCM105: herstellbar gemäß EP0338410B1
- MM28 = CGSC* #5892: Nagahari et al. 1985
- RV308 = ATCC** 31608; EP0677109B1
- RR1 : ATCC** 31434: Nagahari et al., 1985
- KG1005 ompT: Wadensten et al., 1991

* käuflich erhältlich über das E. coli Genetic Stock Center CGSC (830 Kline Biology Tower, MCD Biology Department, 266 Whitney Ave., PO box 208103, Yale University, New Haven,
** käuflich erhältlich über LGC Promochem, Mercatorstr. 51, 46485 Wesel, Deutschland

Das sekretierte Protein ist ein rekombinantes Protein, welches von der Wirtszelle produziert und sekretiert wird. Bevorzugt wird die Sekretion des Proteins durch Fusion seines Gens an eine Signalsequenz ermöglicht. Bevorzugt handelt es sich bei dem produzierten Protein um ein Protein, das Verwendung in technischen Ansätzen findet oder um ein Protein, das als Pharmawirkstoff eingesetzt wird (Biologics, Biopharmazeutikum).

Die Identifizierung von das produzierte Protein verunreinigenden Wirtsproteinen erfolgt nach dem Fachmann bekannten Methoden, etwa durch Auftrennung des zellfreien Überstandes nach Kultivierung in einem SDS-Polyacrylamid-Gel und durch anschließende Analyse der einzelnen Banden durch N-terminale Sequenzierung oder Peptide-Fingerprinting.

Beispiele von Proteinen, die in E. coli das sekretierte Produkt verunreinigen, sind: OppA, DppA, OmpA, YddS, FliC, PhoA, PhoS, außerdem jegliche Kombination dieser Proteine.

- **Oligopeptid-Bindeprotein OppA:** Swiss Prot # P23843 61 kDa, eines der Haupt-Proteine im Periplasma, Komponente der Oligopeptid-Permease (Bindeprotein-abhängiges Transportsystem), bindet Peptide bis zu 5 AA mit hoher Affinität, wird von SecB gebunden, besitzt auch Chaperon-Funktion, beteiligt an der Aufnahme von Aminoglycosid-Antibiotika.
- **Dipeptid-Bindeprotein DppA:** Swiss Prot # P23847 61 kDa, periplasmatisch, Dipeptid-Bindungsprotein eines Transportsystems, nötig für Peptid-Chemotaxis, Chaperon-ähnliche Funktion (in Rhodobacter).
- **Outer membrane protein Omp3a = OmpA:** NCBI # NP 286832 37 kDa, in äußerer Membran lokalisiert, nötig für die Wirkung der Colicine K und L, Stabilisierung während der Konjugation, Rezeptor für Phagen, Porin mit niedrigerer Permeabilität für kleine Solute.
- **Flagellin FliC:** Swiss Prot # P04949, 51 kDa, Untereinheit der Flagellen
- **putatives Hemin-Bindeprotein YddS:** Swiss Prot # Q8XAU4 57 kDa, putatives Dipeptid-Transport-Protein.
- Alkalische Phosphatase PhoA: E.C.3.1.3.1, 49 kDa, periplasmatisches Protein, welches die Orthophosphat-Monoester-Spaltung katalysiert.
- **Phosphat-Bindeprotein PhoS:** 37 kDa, periplasmatisches Protein; Komponente des PTS-Phosphat-Aufnahmesystems.

Die alkalische Phosphatase PhoA und das Phosphat-Bindeprotein PhoS sind beide an der Phosphat-Versorgung von E. coli beteiligt.

Danach wird in den Wirtszellen die Expression der Gene dieser Proteine verringert oder verhindert. Da diese Proteine natürlich in den Bakterien eine physiologische Rolle erfüllen, ist es für den Fachmann völlig unerwartet, dass die drastisch verminderte Bildung dieser Proteine mit einem Überleben der Zellen und einem effektiven Stoffwechsel, wie er für die Überproduktion von Proteinen nötig ist, vereinbar ist.

Methoden, wie die Expression von Genen verringert oder verhindert wird, so dass die speziellen, in der Ausgangszelle von diesem Gen codierten Wirtsproteine weniger oder nicht mehr produziert werden, sind bekannt.

Die Expression eines der Gene kann zum Beispiel durch eine der folgende Maßnahmen verringert oder verhindert werden:

- Abschwächung des dem Gen zugehörigen Promotors durch geeignete Basensubstitutionen
- Inaktivierung/Veränderung eines für die Expression nötigen Transkriptionsaktivators
- Abschwächung von Translationsstartsignalen (z. B. Ribosomenbindestelle, Startcodon) durch geeignete Basensubstitutionen
- Entfernung von mRNA-stabilisierenden Regionen des Gens
- Überexpression von für spezifische Antisense-RNA codierenden DNA-Bereichen
- Deletion des gesamten Gens oder zumindest eines Teils davon
- Zerstörung des Gens durch Insertion von beispielsweise einer Antibiotikumsresistenzkassette
- Einführung von Leserasterverschiebungen in das entsprechende
  Gen aufgrund von Nukleotid-Deletionen oder -Insertionen

Methoden zum Austausch einer beliebigen chromosomalen DNA-Sequenz gegen eine zwar homologe, aber durch Baseninsertionen,

- deletionen oder -substitutionen veränderte Sequenz sind dem Fachmann bekannt. So kann in Escherichia coli beispielsweise das von Link et al. (1997) beschriebene System verwendet werden, um mittels integrativer Plasmide über den Mechanismus der homologen Rekombination die chromosomale Wildtyp-Sequenz des Gens für ein verunreinigendes Protein gegen ein mutiertes Allel auszutauschen.

Bevorzugt ist die Einführung einer Deletion in ein Gen für ein verunreinigendes Wirtsprotein. Dies kann dadurch erreicht werden, dass das Gen nach der Amplifikation mittels PCR unter Einsatz von spezifischen Primern, die das komplette Gen erfassen, zunächst in einen Plasmid-Vektor (z. B. pUC18, pBR322, pACYC184) kloniert wird. Durch Restriktion des so erhaltenen Plasmids mit geeigneten Restriktionsendonukleasen, die nur im Bereich des Gens schneiden, können interne Regionen des Gens entfernt werden. Auf diese Weise kann nach Religation des restringierten Plasmids eine interne Deletion in das Gen eingeführt werden. Alternativ zur Religation des im Gen restringierten Plasmids kann auch eine Antibiotikumsresistenzkassette in das Gen kloniert werden.

Beispielsweise kann die Deletion eines Gens mit Hilfe des $\lambda$ -Red Rekombinase-System von Datsenko und Wanner (2000, PNAS, 97 (12), S. 6640 - 6645) erfolgen.

Die Erfindung betrifft ferner ein Verfahren zur fermentativen Produktion eines rekombinanten Proteins mittels eines Bakterienstammes enthaltend ein rekombinantes Gen kodierend für das rekombinante Protein in einem Fermentationsmedium, welches dadurch gekennzeichnet ist, dass ein erfindungsgemäßer Mikroorganismenstamm in dem Fermentationsmedium kultiviert wird und das Fermentationsmedium nach der Fermentation von den Zellen des Bakterienstammes abgetrennt wird.

Vorzugsweise wird nach dem Abtrennen des Fermentationsmediums das rekombinante Protein aus dem Fermentationsmedium aufgereinigt. Durch das erfindungsgemäße Verfahren wird die Aufreinigung des produzierten rekombinanten Proteins aus dem Fermentationsmedium vereinfacht.

Vorzugsweise wird das Gen, welches für das zu produzierende Protein codiert, mit in diesem Wirtsorganismus funktionellen Expressionssignalen versehen (Promotor, Transkriptions-, Translationsstart, Ribosomenbindestelle).

Zusätzlich wird das für das zu produzierende Protein codierende Gen mit einer Signalsequenz verknüpft, welche bewirkt, dass das zu produzierende Protein zunächst als Fusion mit dem von der Signalsequenz codierten Signalpeptid produziert wird. Dieses Signalpeptid bewirkt die Sekretion des produzierten Proteins.

Als Signalsequenz werden beispielsweise phoA, ompA, pelB, ompF, ompT, lamB, malE, Staphylococcal protein A, stII verwendet.

Die Sekretion des produzierten Proteins erfolgt beispielsweise durch den sec-Apparat der Zelle. Nach erfolgter Sekretion in das Periplasma wird von einer Signalpeptidase (z. B. LepB bei E. coli) das Signalpeptid abgespalten und es entsteht das gewünschte Protein.

Das Gen für das zu produzierende Protein inklusive der Expressions- und Sekretionssignale wird in die Wirtszelle eingebracht. Dies erfolgt auf einem Vektor, z. B. mit einem Plasmid wie etwa einem Abkömmling eines bekannten Expressionsvektors wie pUC18, pBR322, pACYC184, pASK-IBA3 oder pET. Das Gen kann auch vom Chromosom der Wirtszelle aus exprimiert werden.

Die Fermentation des Bakterienstammes zur erfindungsgemäßen Produktion von Proteinen erfolgt vorzugsweise in einem Vollmedium oder Minimalsalzmedium. Diese Medien sind aus der Literatur bekannt.

Als Kohlenstoffquelle können prinzipiell alle verwertbaren Zucker, Zuckeralkohole, organische Säuren bzw. deren Salze, Stärkehydrolysate, Melasse oder andere Stoffe verwendet werden. Dabei werden bevorzugt Glucose oder Glycerin eingesetzt. Auch ist eine kombinierte Fütterung mehrerer verschiedener Kohlenstoffquellen möglich. Als Stickstoffquellen können Harnstoff, Ammoniak und seine Salze, Nitratsalze sowie andere N-Quellen dienen. Zu den möglichen Stickstoffquellen gehören auch komplexe Aminosäuregemische wie Hefeextrakt, Pepton, Malzextrakt, Sojapepton, Casaminosäuren, Maisquellwasser (Corn Steep Liquor) und NZ-Amine (z. B. Kerry Bio-Science, Chicago, USA).

Des weiteren können dem Medium weitere Komponenten zugegeben werden wie Vitamine, Salze, Hefeextrakt, Aminosäuren und Spurenelemente, durch die das Zellwachstum verbessert wird.

Die Inkubation des Stammes erfolgt vorzugsweise unter aeroben Kultivierungsbedingungen über einen Zeitraum von 16 - 150 h und im Bereich der für den jeweiligen Stamm optimalen Wachtumstemperatur.

Als optimaler Temperaturbereich werden 15 - 55°C bevorzugt. Besonders bevorzugt ist eine Temperatur zwischen 30 und 37°C.

Die Anzucht des Stammes kann im Schüttelkolben oder Fermenter erfolgen, wobei keine Beschränkungen hinsichtlich Volumen gegeben sind. Die Anzucht kann im Batch-Verfahren, im Fed-Batch- oder in einem kontinuierlichen Verfahren erfolgen.

Die Aufreinigung von Proteinen aus dem Periplasma bzw. dem Kulturmedium kann nach dem Fachmann bekannten Verfahren, wie Zentrifugation des Mediums zur Abtrennung der Zellen und anschließende chromatographische Reinigung, Komplexierung, Filtration oder Fällung des Proteins erfolgen.

Die folgenden Beispiele dienen der weiteren Erläuterung der Erfindung.

**Beispiel 1: Identifizierung von verunreinigenden Wirtsproteinen**

Folgende aus der Literatur bekannten, allgemein zugänglichen und käuflich erhältlichen E. coli-Sekretionsstämme wurden in LB-Medium in 100 ml-Erlenmeyer-Kolben für 48 h bei 30°C kultiviert:

- BLR: Ray et al. 2002, erhältlich über Novagen
- K802 = CGSC* #5610: Yang et al., 1998
- WCM105: herstellbar gemäß EP0338410B1
- MM28 = CGSC* #5892: Nagahari et al. 1985
- RV308 = ATCC** 31608; EP0677109B1
- RR1 : ATCC** 31434: Nagahari et al., 1985
- KG1005 ompT: Wadensten et al., 1991

***** erhältlich über das E. coli Genetic Stock Center CGSC (830 Kline Biology Tower, MCD Biology Department, 266 Whitney Ave., PO box 208103, Yale University, New Haven, CT 06520-8193,
****** erhältlich über: LGC Promochem, Mercatorstr. 51, 46485 Wesel, Deutschland

Danach wurden die Zellen durch Zentrifugation für 10 Min. bei 13000 g abgetrennt. Vom Überstand (Medium) wurden jeweils 30 $\mu$l mit SDS-Probenpuffer (5 x SDS-Probenpuffer: 125 mM Tris pH 6,8; 10 % Glycerol; 0,5 % SDS; 0,05 % Bromphenol-Blau; 5 % ß-Mercaptoethanol) versetzt und in einem 12 % NuPAGE® Bis-Tris-Gel (Invitrogen Cat.No. NP0341) mit 1x MES-haltigem Running Buffer (20x MES - Running Buffer, Invitrogen Cat.No. NP0002) aufgetrennt (Elektrophorese-Parameter: 40 Min. bei 200 V). Danach wurde das Gel mit Coomassie Blue Färbelösung (1 Tablette PlusOne Coomassie Tabletts, Phast Gel Blue R-350 (Amersham 17-0518-01) in 80 ml $H_2O$ lösen, + 120 ml Methanol, + 200 ml Essigsäure 20 %) 1 h gefärbt und in Entfärbelösung (300 ml Methanol, 100 ml Eisessig, 600 ml vollentsalztes $H_2O$) entfärbt. Nach Waschen in vollentsaltztem Wasser wurden prominente Banden auf dem Gel identifiziert (s. Fig. 1). Diese werden durch das Rohprodukt verunreinigende Proteine verursacht. Die entsprechenden Banden wurden ausgeschnitten und abwechselnd mit Acetonitril und 50 % Ammomiumbicarbonatpuffer (pH 8.5) gewaschen. Bei Bedarf erfolgte die Reduzierung der Disulfidbrücken mit DTT und Jodacetamid. Nach Proteolyse mit Trypsin wurden die entstandenen Peptide mittels MALDI-TOF Massenspektrometrie gemessen. Durch Vergleich mit theoretisch errechneten Massen (erwartete Peptide) bzw. Datenbank-Einträgen wurden die untersuchten Proteine identifiziert.

Es wurden sieben auffällige Proteine identifiziert:

- **Oligopeptid-Bindeprotein OppA:** Swiss Prot # P23843
- **Dipeptid-Bindeprotein DppA:** Swiss Prot # P23847
- **Outer membrane protein Omp3a = OmpA:** NCBI # NP_286832
- **Flagellin FliC:** Swiss Prot # P04949
- **putatives Hemin-Bindeprotein** YddS: Swiss Prot # Q8XAU4
- **Alkalische Phosphatase PhoA:** E.C.3.1.3.1 Swiss Prot # P00634
- **Phosphat-Bindeprotein PhoS Swiss prot** Swiss Prot # P06128

**Beispiel 2. Deletion von Genen für verunreinigende Wirtsproteine**

Die Deletion eines großen internen Bereichs der jeweiligen Gene in den jeweiligen Stämmen erfolgte mit dem $\lambda$-Red Rekombinase-System von Datsenko und Wanner (2000, PNAS, 97 (12), S. 6640 - 6645). Dazu wurde zunächst der zu entfernende Genbereich gegen eine Chloramphenicol-Resistenzkassette ausgetauscht, welche anschließend durch Einsatz der Hefe FLP Rekombinase und spezielle "FRT" Flanken an der Kassette wieder eliminiert werden konnte.

Die Deletion wurde folgendermaßen durchgeführt:

**PCR:**

- Template:

  Als Template wird pKD3 (Chloramphenicol-Resistenz; erhältlich über das E. coli Genetic Stock Center CGSC als CGSC #7631) eingesetzt.

- Oligos:

  <u>Forward Oligo:</u> 36 - 50 bp homologer Sequenz vom Anfang des zu entfernenden Gens + 20 bp homologer Plasmidsequenz:

5 - 36 - 50 bp chrom. -GTG TAG GCT GGA GCT GCT TC - 3' (SEQ. ID NO 1)

Reverse Oligo: 36 - 50 bp homologe Sequenz vom Ende des zu entfernenden Gens + 20 bp Plasmidsequenz (Gegenstrang):

5'- 36 - 50 chrom. - CAT ATG AAT ATC CTC CTT AG - 3' (SEQ. ID NO 2)

- Polymerase:

  Pfu oder Taq

- Produktlänge:

  pKD3: 1,1 kb

**Reinigung des PCR-Produktes:**

Das PCR Produkt wird gereinigt (z. B. über Qiapräp Säulchen) und mit DpnI verdaut (2 h 37°C in zugehörigem Puffer), danach nochmals gereinigt und in 30 μl dest. Wasser eluiert (= PCR-Präp).

**Präparation der elektrokompetenten Zellen:**

● pKD46 = λ Red Rekombinase Expressionsplasmid (Arabinoseinduzierbar) erhältlich über das E. coli Genetic Stock Center CGSC als CGSC #7739, Amp-Resistenz, temperatursensitiver Origin: alle Schritte bei max. 30°C

● Transformation des Plasmids in den zu verändernden Ziel-Stamm (Expression/Inkubation bei 30°C)

● Aus Ziel-Stamm + Plasmid kompetente Zellen für Elektroporation herstellen.

● Medium für die Anzucht der Zellen: SOB Medium:

| | |
|---|---|
| 20 g/l | Trypton |
| 5 g/l | Hefeextrakt |
| 0,5 g/l | NaCl |
| 2,5 g/l | KCl |
| 10 mM | MgCl |
| + 0,2 % | Arabinose |
| + 100 mg/l | Ampicillin |

● Herstellung Elektroporation-kompetenter Zellen:

- Anzucht bei 30°C
- Ernte der Zellen bei $OD_{600}$ ~ 0,6
- ca. 100fach aufkonzentrieren und 3 x mit eiskaltem Glycerin (10 %) waschen

**Rekombination:**

- 10 μl des PCR-Präps (ca. 10 - 100 ng gereinigte PCR-DNA) werden in die elektrokompetenten Zellen transformiert
- Phänotypische Expression: Transformationsansatz in 1 ml LB aufnehmen und 1 h bei 37°C inkubieren) und auf LBcam Platten selektiert. Inkubation bei 37°C.

**Curing von pKD46** (CGSC #7739):

- Ein so erhaltener Klon mit Resistenzkassette wird auf 2 LB Platten ausgestrichen und parallel bei 37°C und 43°C bebrütet.
- Von diesen Platten werden Einzelkolonien auf LB amp (LB mit 100 mg/l Ampicillin) und LB ausgestrichen und bei 30°C inkubiert.
- Ca. 8 Klone davon werden auf LB cam (LB mit 30 mg/l) (37°C) gereinigt.

**PCR Kontrolle der Klone:**

- Mit geeigneten Oligos (s. u. und Datsenko und Wanner, 2000) wird überprüft, ob die Resistenzkassette tatsächlich an Stelle des zu entfernenden Gens im Chromosom sitzt.

**Entfernung der chromosomalen Antibiotikumsresistenzkassette:**

- pCP20 (erhältlich über das E. coli Genetic Stock Center CGSC als CGSC #7629) = "FLP"-Rekombinase Expressionsplasmid, Amp-Resistenz, temperatursensitiver Origin: alle Schritte bei max. 30°C.
- Ein amp-sensitiver Klon (cam-resistenter aus dem Curing!) wird mit pCP20 transformiert, Selektion auf LB amp bei 30°C.
- 8 Transformanten werden auf LB gereinigt, Inkubation bei **37°C,**
- Einzelkolonien werden nochmals auf LB ausgestrichen und bei **43°C** inkubiert.
- Davon werden ca. 12 Klone auf cam und amp Sensitivität getestet.
- amp/cam-sensitive Klone haben die Resistenzkassette und pPC20 verloren.

⇒ Deletionsklon **ohne** integrierter Cam-Kassette.

Im speziellen wurden für die Konstruktion der einzelnen Deletionsmutanten folgende Oligos verwendet und folgende PCR-Produkte erhalten:

**Deletion von OmpA**

Primer für PCR:

OmpA5:

CCAGTACCAT GACACTGGTT TCATCAACAA CAATGGCCCG ACCCATGAAA ACCAA CAT ATG AAT ATC CTC CTT AG (SEQ ID NO. 3)

OmpA6:

GACCCTGGTT GTAAGCGTCA GAACCGATGC GGTCGGTGTA ACCCAGAACA ACTAC GTG TAG GCT GGA GCT GCT TC (SEQ ID NO. 4)

-> PCR mit pKD3 (erhältlich über das E. coli Genetic Stock Center CGSC als CGSC #7631): Produkt: 1114 bp; 598 bp von ompA werden bei Rekombination ersetzt durch 1014 bp Cam-Resistenz Überprüfung der Integration/Deletion mit Primern:

OmpA3: GACAGCTATCGCGATTGCAG (SEQ ID NO. 5)
OmpA4: GCTGAGTTACAACGTCTTTG (SEQ ID NO. 6)

Produkte: WT: 1022 bp; Insertionsmutante: 1486 bp; Deletionsmutante: ca 430 bp

Wie beschrieben wurden die folgenden Stämme hergestellt:

- BLRΔompA
- K802ΔompA

- WCM105ΔompA
- MM28ΔompA
- RV308ΔompA
- RR1ΔompA
- KG1005ompTΔompA

Deletion von OppA

Primer für PCR:

OppA5:

CACTGGCGGA AAAACAAACA CTGGTACGTA ACAATGGTTC AGAAGTTCAG TCATT CAT ATG AAT ATC CTC CTT AG (SEQ ID NO. 7)

OppA6:

CATTCACGTA GTAATAAACA GGAACAATGG CCGAATCCTT ATCCAGCTGT TGTTC GTG TAG GCT GGA GCT GCT TC (SEQ ID NO. 8)

-> PCR mit pKD3: Produkt: 1114 bp; 1331 bp von oppA werden bei Rekombination ersetzt durch 1014 bp Cam-Resistenz Überprüfung der Integration/Deletion mit Primern:

OppA3: GCG GAT CTT TGC CGG TAT AG (SEQ ID NO. 9)
OppA4: GAC CAA CAT CAC CAA GAG AA (SEQ ID NO. 10)

Produkte: WT: 1587 bp; Insertionsmutante: 1270 bp; Deletionsmutante: ca. 260 bp
Es entstanden folgende Stämme:

- BLRΔoppA
- K802ΔoppA
- WCM105ΔoppA
- MM28ΔoppA
- RV308ΔoppA
- RR1ΔoppA
- KG1005ompTΔoppA

**Deletion von DppA**

Primer für PCR:

DppA1:

AAGGGTTTAACCCGCAGCTGTTTACCTCCGGCACCACCTATGACGCCTCT CAT ATG AAT ATC CTC CTT AG (SEQ ID NO. 11)

DppA2:

TGCCGGAGCCTGATCGTGCATCACCACCTGCGCTTGTTTGTACAGTTCAA GTG TAG GCT GGA GCT GCT TC (SEQ ID NO. 12)

-> PCR mit pKD3: Produkt: 1114 bp; 1381 bp von dppA werden bei Rekombination ersetzt durch 1014 bp Cam-Resistenz Überprüfung der Integration/Deletion mit Primern:

DppA3: GTCAGGGATGCTGAAGCTTG (SEQ ID NO. 13)
DppA4: TGTTTGCCTAATGGATCAAC (SEQ ID NO. 14)

Produkte: WT: 1587 bp; Insertionsmutante: 1193 bp; Deletionsmutante: ca. 566 bp

Es entstanden folgende Stämme:

- BLRΔdppA
- K802ΔdppA
- WCM105ΔdppA
- MM28ΔdppA
- RV308ΔdppA
- RR1ΔdppA
- KG1005ompTΔdppA

**Deletion von FliC**

Primer für PCR:

FliC1:

TGCGTATTAACAGCGCGAAGGATGACGCAGCGGGTCAGGCGATTGCTAAC CAT ATG AAT ATC CTC CTT AG (SEQ ID NO. 15)

FliC2:

GGAGTTACCGGCCTGCTGGATGATCTGCGCTTTCGACATATTGGACACTT GTG TAG GCT GGA GCT GCT TC (SEQ ID NO. 16)

-> PCR mit pKD3: Produkt: 1114 bp; 1334 bp von fliC werden bei Rekombination ersetzt durch 1014 bp Cam-Resistenz Überprüfung der Integration/Deletion mit Primern:

FliC3: TATCAACAAGAACCAGTCTGC (SEQ ID NO. 17)
FliC4: AGACAGAACCTGCTGCGGTA (SEQ ID NO. 18)

Produkte: WT: 1432 bp; Insertionsmutante: 1112 bp; Deletionsmutante: ca. 110 bp

Es entstanden folgende Stämme:

- BLRΔfliC
- K802ΔfliC
- WCM105ΔfliC
- MM28ΔfliC
- RV308ΔfliC
- RR1ΔfliC
- KG1005ompTΔfliC

**Deletion von YddS**

Primer für PCR:

YddS1:

ATTGGTAAGGCCGCCGATCCACAAACCCTCGACCCGGCGGTAACAATAGA CAT ATG AAT ATC CTC CTT AG (SEQ ID NO. 19)

YddS2:

ACAGGTACACATAAGCAGCGTCATCAATGACGATTTTCTGTGCCTGCTGG GTG TAG GCT GGA GCT GCT TC (SEQ ID NO. 20)

-> PCR mit pKD3: Produkt: 1114 bp; 1350 bp von fliC werden bei Rekombination ersetzt durch 1014 bp Cam-Resistenz Überprüfung der Integration/Deletion mit Primern:

YddS3: ATTGCTCGCGCTCGTCCTTG (SEQ ID NO. 21)
YddS4: CCTGTTCCAGCATGGGATTG (SEQ ID NO. 22)

Produkte: WT: 1432 bp; Insertionsmutante: 1156 bp; Deletionsmutante: ca. 160 bp

Es entstanden folgende Stämme:

- BLRΔyddS
- K802ΔyddS
- WCM105ΔyddS
- MM28ΔyddS
- RV308ΔyddS
- RR1ΔyddS
- KG1005ompTΔyddS

**Deletion von PhoA und PhoS**

Vorgehen war analog.

Es entstanden folgende Stämme:

- BLRΔphoA
- K802ΔphoA
- WCM105ΔphoA
- MM28ΔphoA
- RV308ΔphoA
- RR1ΔphoA
- KG1005ompTΔphoA
- BLRΔphoS
- K802ΔphoS
- WCM105ΔphoS
- MM28ΔphoS
- RV308ΔphoS
- RR1ΔphoS
- KG1005ompTΔphoS

**Beispiel 3: Erzeugung von Mehrfach-Deletionsmutanten**

Bei der Erzeugung von Mehrfachmutanten wurden in einige der in Beispiel 2 erzeugten Stämmen schrittweise nach dem im Beispiel 2 beschriebenen Vorgehen auch die übrigen 6 identifizierten Gene deletiert.

Es entstanden unter anderem folgende Stämme:

- K802oppAΔfliC
- BLRΔphoAΔoppA
- BLRΔphoAΔoppAΔyddSΔphoAΔphoS
- BLRΔphoAΔyddS
- BLRΔphoAΔoppAΔyddSΔfliC
- WCM105ΔoppAΔompA
- WCM105ΔompAΔfliC
- WCM105ΔoppAΔphoA
- WCM105ΔoppAΔyddS
- WCM105ΔfliCΔyddS
- WCM105ΔoppAΔfliC
- WCM105ΔoppAΔfliCΔyddS
- WCM105ΔoppAΔfliCΔyddSΔphoA

**Beispiel 4: Charakterisierung der Deletionsmutanten bezüglich Wachstum**

Das Wachstum der verschiedenen Stämme bei 30°C in LB mit 1 % Glucose wurde untersucht, indem die OD bei 600 nm nach 24 h und 48 h Wachstum gemessen wurde.

Alle Stämme zeigten normales Wachstum, d. h. das Wachstum war im Vergleich zum Ausgangsstamm nicht eingeschränkt. Die Deletionen haben also keinen negativen Effekt auf die Lebensfähigkeit und das Wachstum der Stämme.

Tabelle 1 zeigt Ergebnisse mit ausgewählten Stämmen:

| Stamm | OD600 (24 h) | OD600 (48 h) |
|---|---|---|
| | | |
| WCM105 | 9,8 | 10,1 |
| WCM105ΔfliC | 10,0 | 10,2 |
| WCM105ΔompA | 9,8 | 10,3 |
| WCM105ΔoppA | 9,9 | 10,1 |
| WCM105ΔyddS | 9,9 | 10,4 |
| WCM105ΔphoA | 9,8 | 10,4 |
| WCM105ΔoppAΔompA | 10,1 | 10,4 |
| WCM105ΔompAΔfliC | 10,2 | 10, 5 |
| WCM105ΔoppAΔphoA | 9,9 | 10,0 |
| WCM105ΔoppAΔfliCΔyddS | 10,0 | 10,2 |
| WCM105ΔoppAΔfliCΔyddSΔphoA | 10,0 | 10,1 |
| K802 | 8, 8 | 12,4 |
| K802ΔoppA | 9,0 | 12,5 |
| K802ΔfliC | 8, 8 | 12,4 |
| K802ΔoppAΔfliC | 9,2 | 12,8 |

**Beispiel 5: Charakterisierung der Deletionsmutanten bezüglich verunreinigende Proteine im Überstand**

Um die verunreinigenden Proteine in den Überständen der erfindungsgemäßen Stämme zu analysieren, wurden von den Anzuchten Aliquots durch SDS-PAGE und Coomassie-Färbung analysiert.

**Beispiel 6: Gesteigerte spezifische Produktion einer Cyclodextrin-Glycosyl-Transferase**

Verschiedene Stämme, erzeugt wie in Beispiel 2 und 3 beschrieben, wurden zur Produktion einer Cyclodextrin-Glycosyl-Transferase eingesetzt. Dazu wurden die Stämme mit Plasmid pCM301 nach gängigen Methoden (z. B. mittels $CaCl_2$-Transformation) transformiert. Dieses Plasmid enthält das Strukturgen der Cyclodextrin-Glycosyl-Transferase aus Klebsiella oxytoca M5al unter Kontrolle des tac-Promotors und ist in EP0220714 beschrieben.

Diese erfindungsgemäßen Stämme werden in 10 ml in LB-Medium mit 1% Glucose bei 30°C angezogen. Bei OD 0,5 wird die Produktion der Cyclodextrin-Glycosyl-Transferase durch Zugabe von IPTG (Isopropylthiogalactosid) ad 0,5 mM induziert.

Im Überstand der Anzuchten der Stämme wurde sowohl der Gesamt-Proteingehalt bestimmt (nach der Bradford-Methode) als auch die Ausbeute an Cyclodextrin-Glycosyl-Transferase durch folgenden Aktivitätstest bestimmt:

Testpuffer: 5 mM Tris-HCl-Puffer > pH 6,5, 5 mM $CaSO_4 . 2 H_2O$ Substrat: 10 %ige Noreduxlösung in Test-Puffer (pH 6,5) Testansatz: 1 ml Substratlösung + 1 ml abzentrifugierter Kulturüberstand (5 Min.,12 000 rpm) + 3 ml Methanol Reaktionstemperatur: 40°C

Enzymtest:

◊ Vortemperieren der Lösungen (ca. 5 Min. bei 40°C)
◊ Zugabe der Enzymlösung zur Substratlösung; rasches Mischen (Whirl-Mixer)

◊ Inkubation für 3 Min. bei 40°C
◊ Stoppen der Enzymreaktion durch Methanol-Zugabe; rasches Mischen (Whirl-Mixer)
◊ Abkühlen des Ansatzes auf Eis (ca. 5 Min.)
◊ Abzentrifugieren (5 Min., 12 000 rpm) und Abpipettieren des klaren Überstandes

◊ Analyse des produzierten CDs mittels HPLC A = G*V1*V2/(t*MG) ( Units / ml ) A = Aktivität
G = Gehalt an CD in mg/l = Testansatz: Flächeneinheiten x $10^4$ / Standardlösung (10 mg/ml)/ Flächeneinheiten
V1 = Verdünnungsfaktor / Testansatz (→ 5)
V2 = Verdünnungsfaktor / Enzymlösung
t = Reaktionszeit in Min. (→ 3)
MG = Molekulargewicht in g/mol (CD → 973)
1 Unit = 1 μMol Produkt / Min.

Tabelle 2 zeigt die gesteigerte spezifische Cyclodextrin-Glycosyltransferase-Ausbeute ausgewählter erfindungsgemäßer Stämme.

| Stamm (jeweils mit pCM301) | Gesamtprotein im Überstand (mg/l) | Produzierte Cyclodextrin-Glycosyl-transferase (U/ml) | Spezifische Cyclodextrin-Glycosyl-transferase-Ausbeute (U/mg) |
|---|---|---|---|
| | | | |
| WCM105 | 503 | 99 | 196,8 |
| WCM105ΔphoA | 490 | 98 | 200, 0 |
| WCM105ΔfliC | 482 | 104 | 215,8 |
| WCM105ΔyddS | 498 | 99 | 198,8 |
| WCM105ΔoppA | 500 | 110 | 220,0 |
| WCM105ΔompA | 470 | 103 | 219, 1 |
| WCM105ΔoppAΔompA | 461 | 101 | 219,1 |
| WCM105ΔompAΔfliC | 470 | 105 | 223,4 |
| WCM105ΔoppAΔphoA | 476 | 102 | 214,3 |
| WCM105ΔoppAΔfliCΔyddS | 464 | 97 | 209,1 |
| WCM105ΔoppAΔfliCΔyddSΔphoA | 451 | 107 | 237,3 |
| K802 | 320 | 67 | 209,4 |
| K802ΔfliC | 307 | 68 | 221,5 |
| K802ΔoppA | 298 | 61 | 204,7 |
| K802ΔoppAΔfliC | 275 | 62 | 225,5 |

**Beispiel 7. Gesteigerte spezifische Produktion von DsbG mit Stämmen erzeugt gemäß Bsp. 2 und 3**

**7.1 Klonierung von dsbG**

Ein Vektor zur Überproduktion von dsbG (Swiss Prot # P77202) wurde folgendermaßen konstruiert:

- PCR mit chromosomaler DNA aus W3110 (ATCC # 27325) als Template und folgenden Primern:

dsbG-fw: GCT CTA GAG GAT CCG AAA AGG ACA AAT TAA TGT TAA AAA AG (SEQ ID NO. 23)

dsbG-rev: CGA ATT CTT ATT TAT TCC CCA TAA TGA TAT TAA G (SEQ ID NO. 24)

- Restriktion des 783 bp PCR-Produkts mit XbaI und EcoRI
- Restriktion von pASK-IBA3 (Fa. IBA, Göttingen) mit XbaI und EcoRI
- Ligation der beiden DNA-Fragmente

Das entstandene Plasmid wurde pASK-dsbG (3961 bp) genannt und enthält das Gen für dsbG inklusive Signalsequenz unter Kontrolle des tet-Promotors.

**7.2 Gesteigerte Produktion von DsbG**

Stämme hergestellt gemäß Bsp. 2 und 3 wurden mit Plasmid pASK-dsbG nach gängigen Methoden (z. B. $CaCl_2$-Transformation) transformiert und die in Tab. 3 genannten erfindungsgemäßen Stämme erhalten.

Der Stamm K802ΔoppA / pASK-dsbG wurden am 26.01.2006 bei der DSMZ (Deutsche Sammlung für Mikroorganismen und Zellkulturen GmbH, D-38142 Braunschweig) unter der Nummer DSM 17899 gemäß Budapester Vertrag hinterlegt.

Der Stamm K802ΔfliC / pASK-dsbG wurden am 26.01.2006 bei der DSMZ (Deutsche Sammlung für Mikroorganismen und Zellkulturen GmbH, D-38142 Braunschweig) unter der Nummer DSM 17898 gemäß Budapester Vertrag hinterlegt.

Die Stämme werden in 10 ml in LB-Medium mit 1 % Glucose und 100 mg/l Ampicillin bei 30°C angezogen. Bei OD 0,5 wird die Produktion von DsbG durch Zugabe von Anhydrotetracyclin ad 0,2 mg/l induziert.

Nach verschiedenen Zeiten wird jeweils zellfreier Überstand auf einem SDS-Polyacrylamidgel aufgetrennt und nach Färbung mit Coomassie Brilliant Blue analysiert. Eine quantitative Auswertung erfolgte nach Einscannen mit Biorad GS-800 Calibrated Densitometer mittels der Quantity One 1-D-Analysis Software (Biorad) im Vergleich zu einem Standard.

Wie Tabelle 3 an einigen Beispielen zeigt, ist die Produktion von DsbG in den erfindungsgemäßen Stämmen im Vergleich zu den Ausgangsstämmen verbessert.

Tabelle 3:

| Stamm (jeweils mit pASK-dsbG) | Anzuchtszeit (h) | DsbG-Ausbeute (mg/l) | Gesamtprotein (mg/l) | spezifische dsbG-Produktion (DsbG/ Gesamtprotein) |
|---|---|---|---|---|
| | | | | |
| WCM105 | 9 | 162 | 432 | 0,38 |
| WCM105 | 24 | 291 | 445 | 0,65 |
| WCM105 | 48 | 285 | 460 | 0,62 |
| WCM105 | 72 | 358 | 542 | 0,66 |
| WCM105ΔoppAΔyddSΔfliC | 9 | 428 | 420 | 1,02 |
| WCM105ΔoppAΔyddSΔfliC | 24 | 415 | 425 | 0,98 |
| WCM1050oppAΔyddSΔfliC | 48 | 450 | 440 | 1,02 |
| WCM105ΔoppAΔyddSΔfliC | 72 | 503 | 451 | 1,12 |
| K802 | 72 | 60 | 76 | 0,79 |
| K802ΔoppA | 72 | 59 | 74 | 0,80 |
| K802ΔfliC | 72 | 60 | 72 | 0,83 |
| K802ΔoppAΔfliC | 72 | 60 | 63 | 0,95 |

## Patentansprüche

1. Mikroorganismenstamm der Art Escherichia coli, der ein Gen kodierend ein rekombinantes Protein sowie ein mutiertes Gen kodierend das Wirtsprotein YddS **putatives Hemin-Bindeprotein**, Swiss Prot # Q8XAU4 57 kDa, putatives Dipeptid-Transport-Protein. umfasst, **dadurch gekennzeichnet, dass** das rekombinante Protein in einer Fermentation sekretiert wird und das mutierte Gen kodierend das Wirtsprotein YddS derart mutiert ist, dass es im Vergleich zu dem dem mutierten Gen zugrunde liegenden Wildtyp-Gen eine derart verminderte Expression des Wirtsproteins bewirkt, dass das durch das mutierte Gen kodierte Wirtsprotein in einer Menge produziert und sekretiert wird, die im Vergleich zu der Produktion und Sekretion in einer Wildtyp-Zelle um 100 %, verringert ist.

2. Mikroorganismenstamm gemäß Anspruch 1, **dadurch gekennzeichnet, dass** das rekombinante Protein in das Fermentationsmedium sekretiert wird.

3. Mikroorganismenstamm gemäß einem der Ansprüche 1 oder 2, **dadurch gekennzeichnet, dass** das rekombinante Protein ein heterologes Protein ist.

4. Mikroorganismenstamm gemäß Anspruch 3, **dadurch gekennzeichnet, dass** er ausgewählt ist aus der Gruppe *BLR, WCM105,* MM28 = *N99 = CGSC #5892*, *K802 = CGSC #5610 = WA802, RV308 = A TCC 31608.*

5. Verfahren zur Herstellung eines Stammes gemäß einem der Ansprüche 1 bis 4, bei dem eine Wirtszelle, die mit einem Gen kodierend ein rekombinantes Protein transformiert ist, in einer Fermentation zur Herstellung des rekombinanten Proteins eingesetzt wird, wobei die Wirtszelle das rekombinante Protein sowie weitere Wirtsproteine in das Fermentationsmedium sekretiert und so ein Rohprodukt enthaltend das rekombinante Protein sowie weitere Wirtsproteine erhalten wird, die weiteren Wirtsproteine charakterisiert werden und die Expression des Gens kodierend das Wirtsprotein YddS im Vergleich zu der Produktion und Sekretion in einer Wildtyp-Zelle um 100% verringert ist.

6. Verfahren zur fermentativen Produktion eines rekombinanten Proteins mittels eines Bakterienstammes enthaltend ein rekombinantes Gen kodierend für das rekombinante Protein in einem Fermentationsmedium, welches **dadurch gekennzeichnet ist, dass** ein Mikroorganismenstamm gemäß einem der Ansprüche 1 bis 4 in dem Fermentationsmedium kultiviert wird und das Fermentationsmedium nach der Fermentation von den Zellen des Bakterienstammes abgetrennt wird.

7. Verfahren gemäß Anspruch 6, **dadurch gekennzeichnet, dass** nach dem Abtrennen des Fermentationsmediums das rekombinante Protein aus dem Fermentationsmedium aufgereinigt wird.

## Claims

1. Microorganism strain of the species Escherichia coli comprising a gene coding for a recombinant protein and a mutated gene coding for the host protein YddS (**putative hemin-binding protein**, Swiss Prot # Q8XAU4 57 kDa, putative dipeptide transport protein), **characterized in that** said recombinant protein is secreted during a fermentation and said mutated gene coding for said YddS host protein has been mutated so as to cause reduced expression of said host protein compared to the wild-type gene on which said mutated gene is based such that the host protein encoded by the mutated gene is produced and secreted in an amount which is reduced by 100% compared to production and secretion in a wild-type cell.

2. Microorganism strain according to Claim 1, **characterized in that** the recombinant protein is secreted into the fermentation medium.

3. Microorganism strain according to either of Claims 1 and 2, **characterized in that** the recombinant protein is a heterologous protein.

4. Microorganism strain according to Claim 3, **characterized in that** said strain is selected from the group consisting of *BLR, WCM105, MM28 = N99 = CGSC #5892, K802 = CGSC #5610 = WA802, RV308 = ATCC 31608.*

5. Process for preparing a strain according to any of Claims 1 to 4, which process comprises employing a host cell transformed with a gene coding for a recombinant protein in a fermentation for producing said recombinant protein, said host cell secreting said recombinant protein and other host proteins into the fermentation medium, thus producing a crude product containing the recombinant protein and other host proteins, characterizing said other host proteins and expression of the gene coding for the YddS host proteins being reduced by 100% compared to production and secretion in a wild-type cell.

6. Process for fermentative production of a recombinant protein by means of a bacterial strain harbouring a recombinant gene coding for said recombinant protein in a fermentation medium, which process is **characterized in that** a microorganism strain according to any of Claims 1 to 4 is cultured in said fermentation medium and, after fermentation, said fermentation medium is removed from the cells of said bacterial strain.

7. Process according to Claim 6, **characterized in that** the recombinant protein is purified from the fermentation medium, after the latter has been removed.

## Revendications

1. Souche de micro-organismes de l'espèce Escherichia coli, qui comprend un gène codant pour une protéine recombinante, ainsi qu'un gène muté codant pour la protéine hôte YddS (protéine putative de liaison à l'hémine, Swiss Prot n° Q8XAU4 57 kDa, protéine putative de transport des dipeptides), **caractérisée en ce que** la protéine recombinante est sécrétée lors d'une fermentation, et le gène muté codant pour la protéine hôte YddS est muté de telle sorte qu'il provoque une expression de la protéine hôte, diminuée par comparaison avec celle du gène de type sauvage qui est à la base du gène muté de façon à ce que la protéine hôte codée par le gène muté soit produite et sécrétée en une quantité telle qu'elle soit diminuée de 100 % par comparaison avec la production et la sécrétion dans une cellule de type sauvage.

2. Souche de micro-organismes selon la revendication 1, **caractérisée en ce que** la protéine recombinante est sécrétée dans le milieu de fermentation.

3. Souche de micro-organismes selon l'une des revendications 1 ou 2, **caractérisée en ce que** la protéine recombinante est une protéine hétérologue.

4. Souche de micro-organismes selon la revendication 3, **caractérisée en ce qu'**elle est choisie dans le groupe consistant en *BLR, WCM105, MM28 = N99 = CGSC #5892, K802 = CGSC #5610 = WA802, RV308 = ATCC 31608.*

5. Procédé de préparation d'une souche selon l'une des revendications 1 à 4, dans lequel une cellule hôte, qui est transformée à l'aide d'un gène codant pour une protéine recombinante, est utilisée dans le cadre d'une fermentation pour produire la protéine recombinante, la cellule hôte sécrétant la protéine recombinante, ainsi que d'autres protéines hôtes, dans le milieu de fermentation, et l'on obtient ainsi un produit brut contenant la protéine recombinante ainsi que d'autres protéines hôtes, les autres protéines hôtes sont caractérisées, et l'expression du gène codant pour la protéine hôte YddS est diminuée de 100 % par comparaison avec la production et la sécrétion dans une cellule de type sauvage.

6. Procédé de production fermentative d'une protéine recombinante à l'aide d'une souche bactérienne contenant un gène recombinant codant pour la protéine recombinante dans un milieu de fermentation, qui est **caractérisé en ce qu'**une souche de micro-organismes selon l'une des revendications 1 à 4 est cultivée dans le milieu de fermentation, et, après la fermentation, les cellules de la souche bactérienne sont séparées du milieu de fermentation.

**7.** Procédé selon la revendication 6, **caractérisé en ce que**, après séparation du milieu de fermentation, la protéine recombinante est purifiée à partir du milieu de fermentation.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 0338410 B1, Ray
- US 5264365 A
- WO 2004035792 A1
- WO 9818946 A
- EP 0677109 B1
- EP 0220714 A

### In der Beschreibung aufgeführte Nicht-Patentliteratur

- **DATSENKO ; WANNER.** *PNAS,* 2000, vol. 97 (12), 6640-6645

SEQUENCE LISTING

<110> Consortium fuer elektrochemische Industrie GmbH

<120> Mikroorganismenstamm zur Produktion von rekombinanten Proteinen

<130> Co10520

<140>
<141>

<160> 24

<170> PatentIn Ver. 2.0

<210> 1
<211> 20
<212> DNA
<213> Artificial Sequence

<220>
<223> PCR-Primer pKD3fw

<400> 1
gtgtaggctg gagctgcttc 20

<210> 2
<211> 19
<212> DNA
<213> Artificial Sequence

<220>
<223> PCR-Primer pKD3rev

<400> 2
catatgaata tcctcctta 19

<210> 3
<211> 75
<212> DNA
<213> Artificial Sequence

<220>
<223> PCR-Primer ompA5

<400> 3

```
ccagtaccat gacactggtt tcatcaacaa caatggcccg acccatgaaa accaacatat 60
gaatatcctc cttag                                                  75
```

<210> 4
<211> 75
<212> DNA
<213> Artificial Sequence

<220>
<223> PCR-Primer ompA6

<400> 4

gaccctggtt gtaagcgtca gaaccgatgc ggtcggtgta acccagaaca actacgtgta 60
ggctggagct gcttc 75

<210> 5
<211> 20
<212> DNA
<213> Artificial Sequence

<220>
<223> PCR-Primer ompA3

<400> 5
gacagctatc gcgattgcag 20

<210> 6
<211> 20
<212> DNA
<213> Artificial Sequence

<220>
<223> PCR-Primer ompA4

<400> 6
gctgagttac aacgtctttg 20

<210> 7
<211> 75
<212> DNA
<213> Artificial Sequence

<220>
<223> PCR-Primer oppA5

<400> 7

cactggcgga aaaacaaaca ctggtacgta acaatggttc agaagttcag tcattcatat 60
gaatatcctc cttag 75

<210> 8
<211> 75
<212> DNA
<213> Artificial Sequence

<220>
<223> PCR-Primer oppA6

<400> 8

cattcacgta gtaataaaca ggaacaatgg ccgaatcctt atccagctgt tgttcgtgta 60
ggctggagct gcttc 75

<210> 9
<211> 20
<212> DNA
<213> Artificial Sequence

<220>
<223> PCR-Primer oppA3

<400> 9
gcggatcttt gccggtatag 20

<210> 10
<211> 20
<212> DNA
<213> Artificial Sequence

<220>
<223> PCR-Primer oppA4

<400> 10
gaccaacatc accaagagaa 20

<210> 11
<211> 70
<212> DNA
<213> Artificial Sequence

<220>
<223> PCR-Primer dppA1

<400> 11

aagggtttaa cccgcagctg tttacctccg gcaccaccta tgacgcctct catatgaata 60
tcctccttag 70

<210> 12
<211> 70
<212> DNA
<213> Artificial Sequence

<220>
<223> PCR-Primer dppA2

<400> 12

tgccggagcc tgatcgtgca tcaccacctg cgcttgtttg tacagttcaa gtgtaggctg 60
gagctgcttc 70

<210> 13
<211> 20
<212> DNA
<213> Artificial Sequence

<220>
<223> PCR-Primer dppA3

<400> 13
gtcagggatg ctgaagcttg 20

<210> 14
<211> 20

<212> DNA
<213> Artificial Sequence

<220>
<223> PCR-Primer dppA4

<400> 14
tgtttgccta atggatcaac 20

<210> 15
<212> DNA
<213> Artificial Sequence

<220>
<223> PCR-Primer fliC1

<400> 15

```
tgcgtattaa cagcgcgaag gatgacgcag cgggtcaggc gattgctaac catatgaata 60
tcctccttag                                                        70
```

<210> 16
<211> 70
<212> DNA
<213> Artificial Sequence

<220>
<223> PCR-Primer fliC2

<400> 16

```
ggagttaccg gcctgctgga tgatctgcgc tttcgacata ttggacactt gtgtaggctg 60
gagctgcttc                                                        70
```

<210> 17
<211> 21
<212> DNA
<213> Artificial Sequence

<220>
<223> PCR-Primer fliC3

<400> 17
tatcaacaag aaccagtctg c 21

<210> 18
<211> 20
<212> DNA
<213> Artificial Sequence

<220>
<223> PCR-Primer fliC4

<400> 18
agacagaacc tgctgcggta 20

<210> 19
<211> 70
<212> DNA
<213> Artificial Sequence

<220>
<223> PCR-Primer yddS1

<400> 19

```
attggtaagg ccgccgatcc acaaaccctc gacccggcgg taacaataga catatgaata 60
tcctccttag                                                        70
```

<210> 20
<211> 70
<212> DNA
<213> Artificial Sequence

<220>
<223> PCR-Primer yddS2

<400> 20

```
acaggtacac ataagcagcg tcatcaatga cgattttctg tgcctgctgg gtgtaggctg 60
gagctgcttc                                                        70
```

<210> 21
<211> 20
<212> DNA
<213> Artificial Sequence

<220>
<223> PCR-Primer yddS3

<400> 21
attgctcgcg ctcgtccttg 20

<210> 22
<211> 20
<212> DNA
<213> Artificial Sequence

<220>
<223> PCR-Primer yddS4

<400> 22
cctgttccag catgggattg 20

<210> 23
<211> 41
<212> DNA
<213> Artificial Sequence

<220>
<223> PCR-Primer dsbGfw

<400> 23
gctctagagg atccgaaaag gacaaattaa tgttaaaaaa g 41

<210> 24
<211> 34
<212> DNA
<213> Artificial Sequence

<220>
<223> PCR-Primer dsbGrev

<400> 24
cgaattctta tttattcccc ataatgatat taag 34